# EUROPEAN PATENT SPECIFICATION

(11) **EP 2 861 763 B1**
(45) Date of publication and mention of the grant of the patent: **26.12.2018**
(21) Application number: 13759573.2
(22) Date of filing: 18.06.2013
(51) Int. Cl.: C12Q 1/68

(54) **COMPUTER-BASED PREDICTOR FOR PROSTATE CANCER**
COMPUTERGESTÜTZTER PRÄDIKTOR FÜR PROSTATAKREBS
PRÉDICTEUR INFORMATIQUE POUR LE CANCER DE LA PROSTATE

(30) Priority: 19.06.2012 IT MI20121066
(43) Date of publication of application: 22.04.2015
(73) Proprietor: Euroclone S.P.A., 20016 Pero, (MI) (IT)
(72) Inventor: VENDRAMIN, Anna, I-34125 Trieste (IT); SACCANI, Andrea, I-20149 Milano (IT); SONEGO, Paolo, I-20016 Pero, Milano (IT); CAPPUCCILLI, Guido, 4123 Allschwil (CH)
(74) Representative: Croce, Valeria
(86) International application number: PCT/IB2013/055004
(87) International publication number: WO 2013/190468

(56) References cited:
- WO-A2-2006/038089
- US-A1- 2009 215 058
- US-B1- 6 821 724
- FEDERICA RIZZI ET AL: "A Novel Gene Signature for Molecular Diagnosis of Human Prostate Cancer by RT-qPCR", PLOS ONE, vol. 3, no. 10, 1 January 2008 (2008-01-01), page e3617, XP055057942, ISSN: 1932-6203, DOI: 10.1371/journal.pone.0003617
- BETTUZZI S ET AL: "MOLECULAR DIAGNOSIS OF HUMAN PROSTATE CANCER (CAP) BY RRT-QPCR DETERMINATION OF GENE EXPRESSION SIGNATURE", EUROPEAN UROLOGY SUPPLEMENTS, XX, XX, vol. 5, no. 14, 1 September 2006 (2006-09-01), page 791, XP027981626, ISSN: 1569-9056 [retrieved on 2006-09-01]
- YOUSEF MALIK ET AL: "Recursive Cluster Elimination (RCE) for classification and feature selection from gene expression data", BMC BIOINFORMATICS, BIOMED CENTRAL, LONDON, GB, vol. 8, no. 1, 2 May 2007 (2007-05-02), page 144, XP021021786, ISSN: 1471-2105, DOI: 10.1186/1471-2105-8-144
- STATNIKOV ALEXANDER ET AL: "A comprehensive comparison of random forests and support vector machines for microarray-based cancer classification", BMC BIOINFORMATICS, BIOMED CENTRAL, LONDON, GB, vol. 9, no. 1, 22 July 2008 (2008-07-22), page 319, XP021031905, ISSN: 1471-2105
- CHAN K Y ET AL: "Gene signature selection for cancer prediction using an integrated approach of genetic algorithm and support vector machine", EVOLUTIONARY COMPUTATION, 2008. CEC 2008. (IEEE WORLD CONGRESS ON COMPUTATIONAL INTELLIGENCE). IEEE CONGRESS ON, IEEE, PISCATAWAY, NJ, USA, 1 June 2008 (2008-06-01), pages 217-224, XP031325143, ISBN: 978-1-4244-1822-0

## Description

It is the object of the present invention a method for diagnosing a prostate cancer, comprising the analysis of data related to the expression of certain genes by predictive algorithms.

Nowadays, prostate cancer represents one of the most common cancer diseases in western countries, and it is expected that the incidence thereof increase in the future.
In the same way as for other cancers, it is by now generally believed that an early diagnosis, i.e., in the first stages of disease development, is crucial to ensure a positive outcome of the treatment. Nowadays, the traditional diagnosis methods comprise the morphological examination of prostate tissue samples. However, such methods have inherent limitations that are not negligible, for example, relating to the experience of the operator setting up and analyzing the sample. On the other hand, molecular diagnosis techniques study the presence and expression of a specific gene pattern, i.e., the pattern of specific genes involved in a disease. These techniques avoid the problem of interpreting the histological preparation; therefore, these methods are more reliable from this viewpoint, although they have to be standardized and implemented so as to be completed in reduced times and accessible to a much more reduced cost. This could be possible, for example, by limiting the number of the so-called marker genes, i.e., genes the expression levels of which have been observed to change in a way which can be directly related to the development of a given disease. However, in doing so, a not very reliable result might be obtained, just because it is based on a reduced pool of assessments. Actually, methods are known, which are based on different possible algorithms, to identify a set of significant genes (the above-mentioned "marker" genes) from a molecular analysis over a large number of genes. However, as noted above, once such set of significant genes has been identified, the problem to ensure that a diagnosis, carried out on such set, leads to sufficiently reliable results, as close as possible to the results achievable through a larger scale analysis, remains unsolved.
On the other hand, it shall be apparent that carrying out a molecular assay on a high number of genes necessarily involves a higher cost.

Therefore, there is the need to balance two different requirements: on one hand, to take into account a sufficient number of marker genes, such as to obtain a highly reliable result, and, on the other hand, to provide a diagnostic tool at competitive costs.

In addition, once a set of known significant genes has been established, the further need arises, for the sake of cost efficiency and procedure simplicity, to pass from the above-mentioned "significant set of marker genes" to an "optimal sub-set", comprising a further reduced number of genes, while being able to provide sufficiently reliable results. Such a need is mainly unmet when using the known solutions.
The prior-art documents of Federica Rizzi et al ("A novel gene signature for molecular diagnosis of human prostate cancer by RT-PCR", PLOS ONE, vol.3, no.10, 1 January 2008 page e3617) and Bettuzzi S et al ("Molecular Diagnosis of human prostate cancer (CAP) by RRT-QPCR determination of gene expression signature", European Urology Supplements, XX,XX, vol 5, no.14, 1 September 2006) disclose a signature of eight marker genes plus two housekeeper genes and their use in the diagnosis or in determining the prognosis of prostate cancer, but do not disclose the use of the Support Vector Machine (SVM) algorithm for the analysis of the expression of said genes.
Prior-art document US 2009/215058 discloses the use of the Support Vector Machine (SVM) algorithm in the context of a selection of a sub-set of predictor genes.

Therefore, the present invention aims to provide a diagnostic method that, in the various embodiments thereof, meets the above-mentioned needs.

### OBJECT OF THE INVENTION

Therefore, in a first aspect, the invention describes a method for the diagnosis of the prostate tumor (or cancer) as per claim 1 and depending claims 2 to 4.

### DESCRIPTION OF THE DRAWINGS

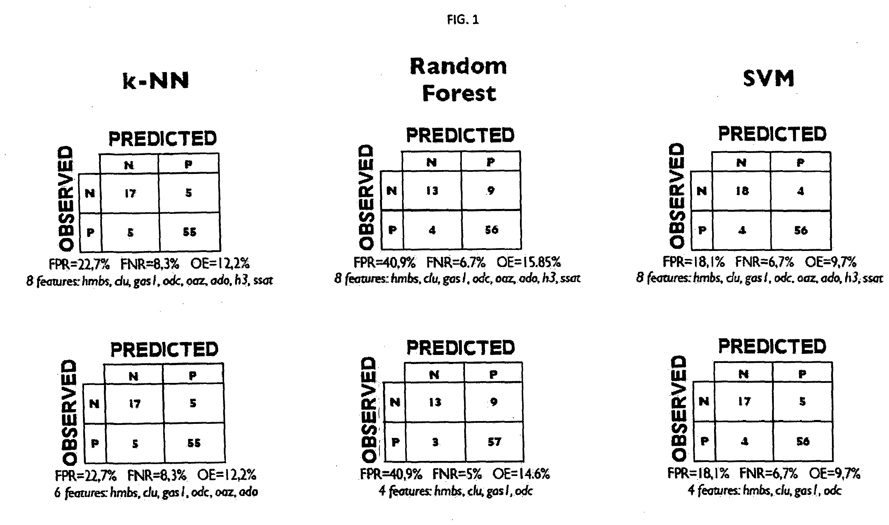

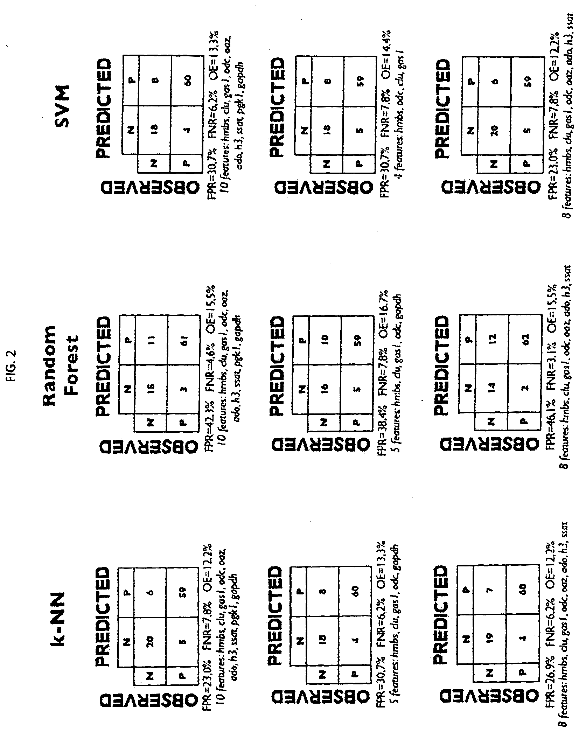

Figs. 1 and 2 represent prediction results (PREDICTED), compared to actually observed results (OBSERVED), quantified in terms of error rate (or "overall error rate" OE), false positive rate (FPR), false negative rate (FNR), obtained by applying several examples of the method according to the invention, and particularly a processing based on three different predictive algorithms (k-NN, Random Forest SVM).

### DETAILED DESCRIPTION OF THE INVENTION

In accordance with a first object of the invention, a method for the diagnosis of prostate cancers, comprising the steps of:
a. determining a plurality of expression values of each one of a group of predictor genes, in an isolated prostate tissue sample,
b. providing to a trained predictive algorithm, operating in vector spaces, an input vector containing in respective vector elements each expression value of said plurality of expression values in said prostate tissue sample to be analyzed;
c. processing the input vector, by the trained predictive algorithm;
d. generating a result representative of said diagnosis for said prostate tissue sample;
   wherein:
   the trained predictive algorithm is Support Vector Machine, and the group of predictor genes is composed of (i) genes coding for HMBS, CLU, GAS I and ODC;
   said method further comprising, before the step a., the step of:
      - training the predictive algorithm, by using, as an input, known expression values in prostate tissue samples corresponding to the diagnosis of prostate cancer.

According to a terminology that is known to those skilled in the algorithm field, the terms "trained predictive algorithm" (i.e., "trained predictor algorithm" i.e., "machine-learning prediction algorithm") are used to indicate, e.g., an algorithm belonging to the class of algorithms capable of estimating an outcome (i.e., usually, estimating a result within a discrete set of possible results), on the basis of known input data, in those cases in which such outcome cannot be determined in an analytic manner based on the input data. The class of trained predictive algorithms that can be exploited in the present invention (as shown in more detail herein below) comprises, for example, parametric algorithms, the results of which depend on adjustable parameters. In such algorithms, the adjustment of the parameters is carried out on the basis of input data, the result of which is already known, and it is carried out by comparing the results generated by the algorithm, as the adjustable parameters change, on a given set of input data, with the respective results that are already known. The step of adjusting the parameters, i.e., the "training", is usually performed as an independent training step, before the use of the algorithm with a predictive function, but it may also be performed continuously during the operation of the algorithm itself.
In an application for diagnostic estimations, the results of the algorithm may be of a binary type, i.e., "true" or "false" (or, in other terms, "healthy" or "sick"), or they can be articulated according to a discrete scale, indicative, for example, of different levels of disease severity.

According to a preferred aspect, the predictor genes (markers) used for the purposes of the present invention belong to a set comprising:
- gene coding for Ornithine decarboxylase (ODC);
- growth arrest-specific gene (GAS 1, also referred to as GAS I);
- gene coding for Clusterin (CLU);
- gene coding for Hydroxymethylbilane synthase (HMBS);

According to an aspect of the invention, the above-mentioned expression of a predictor gene is a value representative of a presence, particularly of the functional aspect, of the corresponding predictor gene in the prostate tissue sample.

According to an aspect of the invention, the prostate tissue sample is pre-isolated by biopsy from a patient and next treated according to the techniques known in the art. In a preferred aspect of the invention, after the withdrawal, the sample is embedded and preserved in paraffin; therefore, it is a "repertoire" or "archival" sample. Of course, before the use for the purposes of the present invention, the paraffin-embedded sample is suitably treated, for example "dewaxed", according to techniques known in the art.
With regard to the analysis of gene expression, this is implemented by a Real Time PCR, according to methods known to those skilled in the art.
Particularly, the following primers are preferably used:
**Gas1 fwd** 5'- CGCACCGTCATTGAGGAC
**Gas1 rev** 5'- CACGCAGTCGTTGAGCAG
**Clu fwd** 5'- CCTCACTTCTTCTTTCCCAAG
**Clu rev** 5'- GTACGGAGAGAAGGGCATC
**Odc fwd** 5'- CAGTCTGTCGTCTCAGTGTG
**Odc rev** 5'- TTCGCCCGTTCCAAAAGGAG
**Hmbs fwd** 5'- CGCTGCATCGCTGAAAGG
**Hmbs rev** 5'- ACGGCTACTGGCACACTG

The above-described sequences represent themselves a further object of the present invention.

According to an aspect of the disclosure the trained predictive algorithm is an algorithm operating in vector spaces, and the above-mentioned step b. (processing) comprises: processing an input vector containing, in respective vector elements, each expression of the plurality of expressions; and generating, depending on such processing, a result representative of the diagnosis and/or prognosis.

According to the invention, the predictive algorithm is: Support Vector Machine (SVM).

According to a further aspect of the invention, the method further comprises, before the step a., the step of training the predictive algorithm, by using as an input the known expression values in the prostate tissue samples, the diagnosis and/or prognosis of which is known, to calibrate adjustable algorithmic parameters and to obtain trained algorithmic parameters.

According to a further aspect of the disclosure, the method further comprises the step of determining the group of predictor genes, within the above-mentioned set of predictor genes, depending on the known expression values of such genes in the prostate tissue samples, the diagnosis and/or prognosis of which is known.

According to a further aspect of the disclosure, the above-mentioned step of determining the group of predictor genes is implemented by an algorithm for dimension reduction of vector spaces.

It shall be noted that the above-mentioned "set of predictor genes" may be referred to a set of significant marker genes, particularly, to a set of marker genes that is known in the medical/diagnostic field considered herein. Preferably, such set is the set already mentioned above.
On the other hand, the above-mentioned "group of predictor genes", forming the base for the diagnosis through a trained predictive algorithm, can be also defined as a "significant optimized subset of predictor genes", i.e., "reduced subset of markers" on which the prediction can be implemented, where the transition from the set of marker genes to the optimized subset (i.e., the "group") of marker genes is carried out, for example, by means of the above-mentioned algorithm for dimension reduction of vector spaces.
In peculiar cases, also comprised in the invention, "group" and "set" of marker genes may coincide.

According to a further aspect of the disclosure, the above-mentioned step of determining the group of predictor genes is comprised in the initial training step.

According to a preferred implementation example of the method, the trained predictive algorithm is Support Vector Machine, and the group of predictor genes is composed of genes coding for HMBS, CLU, GAS I, ODC.

It shall be noted that the two examples set forth above, with reference to the group of predictor genes, is not to be intended as limiting. In fact, as already noticed, in an embodiment of the method of the present disclosure the group of predictor genes is determined by an algorithm for dimension reduction of vector spaces, and therefore such group may also be different from the mentioned ones.

Therefore, in the method of the present disclosure a diagnostic result (for example, "sick" - "healthy") on a tissue sample may be obtained, through a trained predictive algorithm, by using as a set of marker genes the entire starting set, or either of the groups (i.e., subsets) of marker genes set forth above, with reference to the two preferred examples mentioned above, or another group of marker genes, provided that it is identified from the initial set by a suitable algorithm for dimension reduction of vector spaces.

Disclosed is also a kit for the diagnosis and/or for prognosis of prostate cancers, configured to implement the above-described method. Particularly, the kit comprises: determination means for determining a plurality of representative information, each piece of information being representative of an expression of each one of a group of predictor genes, in a prostate tissue sample; and processing means operatively connectable to the determination means to receive the plurality of representative information of the plurality of expressions, the processing means being configured to process the information representative of the plurality of expressions by means of a trained predictive algorithm, to obtain a result representative of said diagnosis and/or prognosis.

The predictor genes disclosed belong to the already mentioned set comprising:
- gene coding for Ornithine decarboxylase (ODC);
- gene coding for Ornithine decarboxylase antizyme (OAZ);
- gene coding for Adenosylmethionine decarboxylase (AdoMetDC);
- gene coding for Spermidine/spermine N(1)-acetyltransferase (SSAT);
- gene coding for Histone H3 (H3);
- growth arrest-specific gene (GAS 1, also referred to as GAS I);
- gene coding for Clusterin (CLU);
- gene coding for Hydroxymethylbilane synthase (HMBS);
- gene coding for Glyceraldehyde 3-phosphate dehydrogenase (GAPDH); and
- gene coding for Phosphoglycerate kinase (PGK1).

Predictor genes (markers) disclosed are:
- gene coding for Ornithine decarboxylase 1 (ODC1);
- gene coding for Ornithine decarboxylase antizyme 1 (OAZ1);
- gene coding for Adenosylmethionine decarboxylase 1 (AMD1);
- gene coding for Spermidine/spermine N(1)-acetyltransferase 1 (SAT1);
- gene coding for histone cluster 1, H3c (HIST1H3C);
- growth arrest-specific gene 1 (GAS 1);
- gene coding for Clusterin (CLU);
- gene coding for Hydroxymethylbilane synthase (HMBS);
- gene coding for Glyceraldehyde 3-phosphate dehydrogenase (GAPDH); and
- gene coding for Phosphoglycerate kinase 1 (PGK1).

According to the present disclosure, the above-mentioned determining means are diagnostic kits, having a structure that is *per se* known, configured to operate on the selected group of predictor genes.

According to the present disclosure, the determination means comprise a data communication interface to the processing means.

According to the present disclosure, the provision of the data coming from the determination means to the processing means is carried out in other known manners, for example, by manually inputting them.

According to the present disclosure, the above-mentioned processing means are a processor, such as a computer, or a personal computer, or a laptop, or a workstation. Such processor is provided with interface and result displaying means, *per se* known; with a *per se* known memory, in which one or more trained predictive algorithms and relative execution programs are stored; and with a processor, *per se* known, for running such programs and algorithms.

In accordance with the present disclosure, the kit according to the invention is configured to implement a corresponding embodiment of the method according to the invention, among those described above.

Herein below, further details about the above-described method will be provided, according to different implementation examples of the invention, and particularly about the predictive algorithms used.

Particularly, the use of algorithms that are configured to operate in vector spaces is provided. For example, different algorithms of this type (*per se* known in the mathematics field) were tested, and proved to be efficient:
- Support Vector Machine (SVM);
- Random Forest;
- k-nearest neighbor (k-NN);
to be used alternatively or in combination one to the other.

As known to those skilled in the art of such algorithms, such algorithms define a "classification" of input samples with respect to possible output results. The k-NN algorithm allows an automatic classification of the samples by allocating a sample to a class based on the majority of the indications ("votes") of its k neighbors, where k is an positive integer (typically not very large).
The Random Forest algorithm is in principle an extension of methods based on classification trees in which a "forest" of classifier trees is generated, each of which proposes a classification for a single element in a given class. By comparing the classification proposals provided by each tree in the forest, the class, to which the element may be allocated, is identified i.e., the class that received most "votes".
The Support Vector Machines (SVM) belongs to the group of the linear separators (such as, for example, the "Artificial Neural Networks") and allow solving classification problems that cannot be solved by a linear separator in a space with a few dimensions, by a mapping, defined by a "kernel" function, of each datum in a space with a higher dimensionality, in which the classification groups can be linearly separated.

Each of the above-mentioned algorithms operates on a vector space; therefore, it may be considered as a "black box", receiving in input a vector and producing, as a function of the input vector, a result among a discrete predefined set of possible results.

In the present disclosure the input vector is representative of the expressions of predictor genes. Particularly, the input vector comprises different vector elements, each being associated to a corresponding predictor gene. Each vector element contains a value representative of the presence of the corresponding gene in the analyzed prostate tissue sample, as detected in the same sample.

According to a preferred implementation example, such representative value may take numerical values, within a continuous or discrete range, corresponding to a measurement of the expression levels of the corresponding predictor gene in the analyzed prostate tissue sample. This advantageously allows to better characterize the prostate tissue, compared to a mere indication of the presence or the absence of the predictor gene, and consequently to improve the prediction accuracy.

However, according to other implementation examples comprised in the disclosure, the representative value can also be simply a binary value (YES/NO, i.e., PRESENT/ABSENT), being associated to the fact that the detected expression level of the corresponding gene is above or below a preset threshold.

The dimension of the vector spaces in which the predictive algorithms operate corresponds to the dimension of the input vectors, thus, to the number of predictor genes belonging to the selected group. According to different implementation examples of the invention, such dimension may be comprised, for example, between 4 and 10.

The predictive algorithm is configured so as to predict first the presence or the absence of the cancer. Moreover, in a further implementation example, it can also be configured to provide a prediction of the cancer severity/progression degree. According to a specific example, in the case that the presence of a cancer is predicted, it is also classified on three Gleason classes (Gleason6, Gleason7, Gleason8) corresponding to as many prognosis degrees.
Therefore, the output of the algorithm (which provides, as already noted, a result among a predefined discrete set of possible results) may simply be a binary result, or it may be non-binary, while being discrete. Therefore, it shall be noticed that the output of the trained predictive algorithm, used as described above, directly provides a result representative of a diagnosis (for example, "healthy" or "sick" output values). Furthermore, in a particular implementation example, such output may also comprise a result representative of the degree of severity/progression of the disease.

Taking now into account the operation modes of the algorithms, it shall be noted that each of the predictive algorithms of the above-mentioned type is characterized by some "adjustable algorithmic parameters", affecting the results. Particularly, the "adjustable algorithmic parameters" are *γ* and *cost* (in SVM), *ntree* and *mtry* (in Random Forest), *k* (in k-NN). Such "adjustable algorithmic parameters" are calibrated during an initial step, the so-called training step (training), to obtain "trained algorithmic parameters", which are then used in the step of actual use of the method for diagnostic/prognostic purposes.

The training step, which is independently provided for each of the algorithms, generally provides to begin from an initial nominal value of the "adjustable algorithmic parameters", to input to the algorithm vectors corresponding to situations for which the results are already known; then, to verify the obtained result; finally to iteratively change the "adjustable algorithmic parameters", until reaching a desired accuracy in the obtained results. Particularly, the training step provides to use, as input vectors, vectors that contain the results detected in a set of prostate tissue samples, the diagnosis and/or prognosis of which is known (in part healthy, in part sick, and, where sick, at different progression stages of the disease).

It shall be noted that the algorithm is referred to as "trained" when the "adjustable algorithmic parameters" were set on the values that produced the more reliable results, during the training step, thus obtaining the "trained algorithmic parameters".

Once that the algorithm has been trained, it can receive in input specific vectors containing data measured in prostate tissue samples, relating to the respective cases for which a prediction is desired, and provides as an output the result of the prediction.

According to an embodiment of the method, the training step is performed by using the so-called "cross-validation" technique. In such a case, the initial data set (the result of which is known) is divided into a training subset (or "training set") and a test subset (or "test set") . The model for the given predictor, i.e., the definition of the adjustable parameters, is built by using each of the "training sets" and assessed on each of the "test sets". For example, in the "10-fold cross-validation", data are divided into ten groups containing approximately a same number of patients, the predictive algorithm is trained on nine groups, and it is validated on the tenth one; then, the obtained accuracy of the model is calculated, and the procedure is repeated until using each group as a "test set" and the remaining ones as "training set". At the end of the above-mentioned procedure, the average of the errors is calculated over the entire series of repetitions that has been carried out.

Data relative to experimental tests that have been performed are given herein below by way of illustrative, non-limiting example. Some of the obtained results are reported in the Figures 1 and 2.

A first series of tests was carried out based on a set of 8 predictor genes (conventionally set forth herein in lower case letters: gas I, h3, ssat, clu, odc, ado, oaz, hmbs). Such first series of tests (the results of which are illustrated in the first row of Fig. 1) comprised tests carried out on a set comprising 82 samples, of which 60 were sick and 22 healthy; and further tests carried out on a set comprising 59 sick samples, on three different degrees of severity (G6, G7, G8) .

A second series of test was carried out based on a set of 10 predictor genes (conventionally indicated herein in lower case letters: gas I, h3, ssat, clu, odc, ado, oaz, hmbs, gapdh, pgK I). Such second series of tests (the results of which are illustrated in the first row of Fig. 2) comprised tests carried out on a set comprising 90 samples, of which 64 were sick and 26 healthy; and further tests carried out on a set comprising 63 sick samples, on three different degrees of severity (G6, G7, G8).

It shall be noted that the sets of predictor genes considered above are derived from practice. It is also opportune to notice that, according to a preferred aspect of the present invention, and unlike most of the traditional approaches, the genes in the set are treated and considered in an equivalent manner, and there is no gene(s) to act as a "reference".

Both the first and the second series of tests were carried out by independently training and then using each of the three predictive algorithms mentioned above.

Training was performed, (as already described) through a calibration of the "adjustable algorithmic parameters" of the algorithms, on the basis of a comparison between the prediction results, as they were provided by the algorithm, and the known results.

Particularly, the so-called "confusion matrices", reported in the Figs. 1 and 2, were compiled, each of which contains, in the rowN-columnN box, the negative cases recognized by the prediction as negative; in the rowP-columnP box, the positive cases recognized by the prediction as positive; in the rowN-columnP box, the negative cases identified by the prediction as positive (FALSE POSITIVE); in the rowP-columnN box, the positive cases identified by the prediction as negative (FALSE NEGATIVE).

Then the results have been observed by dividing them into EXACT, FALSE POSITIVE, FALSE NEGATIVE, and by synthesizing them through the OE (overall error rate), FPR (false positive rate) and FNR (false negative rate) parameters.

The quantitative results set forth in the Figs. 1 and 2 are under many aspect self-evident. However, some remarks are reported herein below.

By taking into account the "confusion matrices" of the first row of Fig. 1, relating to the first series of tests, it shall be observed that:
- through the k-NN algorithm (with a trained parameter k=3), an overall error rate of 12.2% is obtained;
- through the Random Forest algorithm (with trained parameters ntree=500, mtry=3), an overall error rate of 15.8% is obtained;
- through the SVM algorithm (with trained parameters *radial basis*, γ=0.06, cost=4), an overall error rate of 9.7% is obtained.
As it will be noticed herein below, however, it is important to assess not only the overall error rate, but also the false positive and false negative rate, aiming to minimize particularly the false negatives.

By considering the "confusion matrices" of the first row of Fig. 2, relating to the second series of tests, it shall be observed that:
- through the k-NN algorithm (with trained parameter k=3), an overall error rate of 12.2% is obtained;
- through the Random Forest algorithm (with trained parameters ntree=850, mtry=2), an overall error rate of 15.5% is obtained;
- through the algorithm SVM (with trained parameters *radial basis*, γ=0.17, cost=4), an overall error rate of 13.3% is obtained.
As it will be noticed herein below, however, it is important to assess not only the overall error rate, but also the false positive and false negative rate, aiming to minimize particularly the false negatives.

A further implementation example is now illustrated in more detail, relating to the already-mentioned aspect related to the reduction of the number of predictor genes to be used, i.e., to the suitable selection of a group of predictor genes, within a known set of predictor genes.

The reduction of the number of predictor genes (i.e., the dimensions of the base of markers), ideally while keeping the prediction accuracy constant, is advantageous first of all because it simplifies the diagnostic method and the relative diagnostic kits; then, because it allows to reduce the number of samples on which the procedures are performed.
Furthermore, in some cases, such reduction may even allow an improvement in the accuracy of the prediction results, reducing the "noise" in the fluctuation of the results generated by the use of a higher number of predictor genes.

Of course, the reduction of the number of predictor genes implies a targeted selection of the genes that have to be a part of the optimal subset (i.e., "group").

In this regard, the characteristic of the present disclosure to use trained predictive algorithms, operating in vector spaces, advantageously allows to apply statistical-mathematical techniques, *per se* known in the mathematical field, for a "reduction of vector spaces", which operate - starting from an initial vector space - independently from the "meaning" of the content of the vectors.

Therefore, according to an implementation example of the disclosure, the training step comprises the further step of determining the group of predictor genes, within a set of predictor genes, by means of an algorithm belonging to the class of the "algorithms for dimension reduction of vector spaces".

For example, the algorithms for dimension reduction of vector spaces may be based on so-called "feature reduction" methods, applied in combination with the trained predictive algorithm. Such reduction algorithm analyzes the results of the predictive algorithm, during the training step, and provides as an output a piece of information indicative of the "weight" that each of the predictor genes of the initial set has to the purposes of the determination of the result.

This provides a kind of rank of predictor genes, from the most relevant to the least relevant, in terms of influence on the prediction result. Starting from such classification, one can proceed by trials and errors, by executing the predictive algorithm with an increasingly reduced number of predictor genes, paying attention to select, for a group containing N predictor genes, the first N genes of the rank determined by the reduction algorithm. The progressive reduction proceeds until obtaining an optimal result, or anyhow a result that is considered as acceptable, according to preset criteria.

Once the optimal number of predictor genes has been identified, then the respective group of predictor genes is selected, the method proceed to train the predictive algorithm and calibrate again, finely, the adjustable algorithmic parameters thereof.

According to a further embodiment of the disclosure, the selection of the group of predictor genes is carried out by using a paradigm of "feature selection", based on the use of the Random Forest algorithm to select the most relevant variables. In this embodiment, a data "random sampling" technique can be used to select the model with a lower error rate according to the variation in the number of the variables.

In the second row of Fig. 1, and in the second and third rows of Fig. 2, results of further series of tests carried out by applying the above-mentioned reduction techniques are set forth.

Particularly, in the second row of Fig. 1, with reference to the first series of tests, it shall be noted that good results are obtained, in terms of error rate, with groups of 4 predictor genes (with Random Forest and SVM) or of 6 predictor genes (with k-NN), as indicated in the figure. Particularly, the following results were obtained:
- k-NN (k=3), 6 predictor genes (indicated in the figure as "*features*"), overall error rate =12.2%
- Random Forest (ntree=500, mtry=1), 4 predictor genes (indicated in the figure as "*features*"), overall error rate =14.6%
- SVM (radial basis, γ=0.5, cost=4), 4 predictor genes (indicated in the figure as "*features*"), overall error rate =9.7%,
which results are comparable to, and sometimes even better than, the results set forth in the first row of Fig. 1, relative to the use of 8 predictor genes.

Some further synthetic observations about the obtained results are set forth herein below.

The best performance (in terms of a lower overall error rate) were obtained by the predictive algorithm SVM trained on 82 samples; such result was validated also in the case of using only 4 predictor genes, suitably selected as indicated above and illustrated in Fig. 1.

Generally, the different applications of the method, with the different predictive algorithms, showed an occurrence of False Positives (FPR) much higher than the occurrence of False Negatives (FNR). As it will be noticed herein below, this is an advantage in the diagnostic field.

In the series of tests carried out, the lowest error rate was obtained by using the predictive algorithm SVM. On the other hand, the lowest false negative rate FNR has been obtained always by using the predictive Random Forest algorithm, which, however, yielded also the worst overall error rate.

The availability of different predictive algorithms, with different advantages and drawbacks, allows to articulate in an optimal way the application of the method of the present invention, in one of the different possible embodiments, according to the criterion used to assess the results.

As set forth above, the method of the present invention allows to meet the need to provide a diagnostic tool that is reliable and available at a cost accessible to the diagnostic laboratories of private and public bodies, as well as to be competitive with other existing tools.

Particularly, it has been noticed that the method is capable of distinguishing with accuracy positive from negative cases.

Furthermore, the method described above shows an occurrence of false positives (i.e., it provides a result indicative of the disease, when the subject is actually healthy) much higher than the false negatives (i.e., subjects diagnosed as healthy, but who, in further assessments, turn out to be sick).
This fact, which is an advantage in the diagnostic field, is not detected by the observation of the overall error rate (OE), but is apparent from the analysis of the Confusion Matrices.

The predictor herein described allows to classify the disease with an acceptable accuracy into 3 possible classes of severity, i.e., identifying prognostic degrees of Gleason 6, Gleason 7, and Gleason 8 class, where the Gleason class is the classification used to describe a prostate cancer taking into account the cytological characteristics of the cells and their organization.

In addition, the method of the present invention proved to be also a valid prognostic tool, for the predictive assessment of the possible development of the disease upon time.

A particular advantage related to the described method is the possibility to use, for the molecular assays, archival (or "repertoire") samples suitably stored, for example in paraffin, without however excluding the use of "fresh" samples.

Advantageously, this allows to follow the evolution of the disease upon time, and allows to carry out very broad statistical and epidemiological analyses.

## Claims

1. A method for the diagnosis of prostate cancers, comprising the steps of:
a. determining a plurality of expression values of each one of a group of predictor genes, in an isolated prostate tissue sample,
b. providing to a trained predictive algorithm, operating in vector spaces, an input vector containing in respective vector elements each expression value of said plurality of expression values in said prostate tissue sample to be analyzed;
c. processing the input vector, by the trained predictive algorithm;
d. generating a result representative of said diagnosis for said prostate tissue sample;
wherein:
the trained predictive algorithm is Support Vector Machine, and the group of predictor genes is composed of (i) genes coding for HMBS, CLU, GAS I and ODC;
said method further comprising, before the step a., the step of:
- training the predictive algorithm, by using, as an input, known expression values in prostate tissue samples corresponding to the diagnosis of prostate cancer.

2. The method according to claim 1, wherein the step a. is carried out by using Real Time - PCR.

3. The method according to the previous claim, wherein in said step a. the following primers are used:
Gas1 fwd 5'- CGCACCGTCATTGAGGAC
Gas1 rev 5'- CACGCAGTCGTTGAGCAG
Clu fwd 5'- CCTCACTTCTTCTTTCCCAAG
Clu rev 5'- GTACGGAGAGAAGGGCATC
Odc fwd 5'- CAGTCTGTCGTCTCAGTGTG
Odc rev 5'- TTCGCCCGTTCCAAAAGGAG
Hmbs fwd 5'- CGCTGCATCGCTGAAAGG
Hmbs rev 5'- ACGGCTACTGGCACACTG

4. The method according to any of the preceding claims, wherein said isolated sample is a sample that, after isolation, was embedded in paraffin and that, before use, is suitably dewaxed and optionally further processed.

## Patentansprüche

1. Verfahren zur Diagnose von Prostatakrebsen, umfassend die Schritte:
a. Bestimmen einer Vielzahl von Expressionswerten eines jeden Gens aus einer Vielzahl von Vorhersage-Genen in einer isolierten Prostatagewebeprobe,
b. Bereitstellen eines geschulten Vorhersage-Algorithmus, welcher in Vektorräumen arbeitet, eines Eingabevektors enthaltend in entsprechenden Vektorelementen jeden Expressionswert der Vielzahl von Expressionswerten in der zu analysierenden Prostatagewebeprobe;
c. Prozessieren des Eingabevektors durch den geschulten Vorhersage-Algorithmus;
d. Erzeugen eines Ergebnisses, welches repräsentativ für die Diagnose der Prostata-Gewebeprobe ist;
wobei
der geschulte Vorhersage-Algorithmus eine Stütz-Vektor-Maschine ist, und die Gruppe der Vorhersage-Gene zusammengesetzt ist aus
(i) Genen kodierend für HMBS, CLU, GAS I und ODC;
wobei das Verfahren vor Schritt a. ferner den Schritt umfasst:
- Schulen des Vorhersage-Algorithmus unter Verwendung, als Eingabe, von bekannten Expressionswerten in Prostatagewebeproben entsprechend zur Diagnose von Prostatakrebs.

2. Das Verfahren nach Anspruch 1, wobei der Schritt a. durch Verwendung von Echtzeit-PCR durchgeführt wird.

3. Das Verfahren nach einem der vorausgehenden Ansprüche, wobei in Schritt a. die folgenden Primer verwendet werden:
Gas1 vor 5'-CGCACCGTCATTGAGGAC
Gas1 rück 5'-CACGCAGTCGTTGAGCAG
Clu fwd 5'-CCTCACTTCTTCTTTCCCAAG
Clu rück 5'-GTACGGAGAGAAGGGCATC
Odc vor 5'-CAGTCTGTCGTCTCAGTGTG
Odc rück 5'-TTCGCCCGTTCCAAAAGGAG
Hmbs vor 5'-CGCTGCATCGCTGAAAGG
Hmbs rück 5'-ACGGCTACTGGCACACTG:

4. Das Verfahren nach einem der vorausgehenden Ansprüche, wobei die isolierte Probe eine Probe ist, welche nach der Isolierung in Paraffin eingebettet wurde und welche vor der Verwendung in geeigneter Weise entwachst und gegebenenfalls weiter verarbeitet wurde.

## Revendications

1. Procédé pour le diagnostic des cancers de la prostate, comprenant les étapes de :
a. détermination d'une pluralité de valeurs d'expression de chaque gène d'un groupe de gènes prédicteurs, dans un échantillon de tissu prostatique isolé,
b. fourniture à un algorithme prédictif entraîné, fonctionnant dans des espaces vectoriels, d'un vecteur d'entrée contenant dans des éléments de vecteur respectifs chaque valeur d'expression de ladite pluralité de valeurs d'expression dans ledit échantillon de tissu prostatique à analyser ;
c. traitement du vecteur d'entrée à l'aide de l'algorithme prédictif entraîné ;
d. production d'un résultat représentatif dudit diagnostic pour ledit échantillon de tissu prostatique ;
dans lequel :
l'algorithme prédictif entraîné est une Machine à Vecteurs de Support (SVM), et le groupe de gènes prédicteurs est composé (i) des gènes codant pour HMBS, CLU, GAS I et ODC ;
ledit procédé comprenant en outre, avant l'étape a., l'étape de :
- entraînement de l'algorithme prédictif en utilisant comme entrée des valeurs d'expression connues dans des échantillons de tissu prostatique correspondant au diagnostic d'un cancer de la prostate.

2. Procédé selon la revendication 1, dans lequel l'étape a. est réalisée en utilisant une PCR en temps réel.

3. Procédé selon la revendication précédente, dans lequel dans ladite étape a., les amorces suivantes sont utilisées :
Gas1 directe 5' - CGCACCGTCATTGAGGAC
Gas1 inverse 5' - CACGCAGTCGTTGAGCAG
Clu directe 5' - CCTCACTTCTTCTTTCCCAAG
Clu inverse 5' - GTACGGAGAGAAGGGCATC
Odc directe 5' - CAGTCTGTCGTCTCAGTGTG
Odc inverse 5' - TTCGCCCGTTCCAAAAGGAG
Hmbs directe 5' - CGCTGCATCGCTGAAAGG
Hmbs inverse 5' - ACGGCTACTGGCACACTG

4. Procédé selon l'une quelconque des revendications précédentes, dans lequel ledit échantillon isolé est un échantillon qui, après isolement, a été incorporé dans de la paraffine et qui, avant utilisation, est déparaffiné de manière appropriée et éventuellement, soumis à un traitement ultérieur.
